# EUROPEAN PATENT SPECIFICATION

(11) **EP 1 840 594 B1**
(45) Date of publication and mention of the grant of the patent: **06.06.2012**
(21) Application number: 07005391.3
(22) Date of filing: 15.03.2007
(51) Int. Cl.: A61B 8/08, G01S 15/89

(54) **Ultrasonic diagnostic apparatus and data analysis and measurement apparatus**
Ultraschalldiagnosevorrichtung und Vorrichtung zur Datenanalyse und -messung
Appareil de diagnostic ultrasonique et appareil d'analyse de données et de mesure

(30) Priority: 28.03.2006 JP 2006088899
(43) Date of publication of application: 03.10.2007
(73) Proprietor: FUJIFILM Corporation, Minato-ku Tokyo 106-8620 (JP)
(72) Inventor: Satoh, Yoshiaki, Ashigarakami-gun Kanagawa 258-8538 (JP)
(74) Representative: Klunker . Schmitt-Nilson . Hirsch

(56) References cited:
- JP-A- 2005 118 314
- US-A1- 2003 083 563
- US-A1- 2005 004 459
- US-B1- 6 322 505

## Description

### BACKGROUND OF THE INVENTION

### Field of the Invention

The present invention relates to an ultrasonic diagnostic apparatus according to the preamble of claim 1. Such apparatus is used for imaging organs and so on within a living body by transmitting and receiving ultrasonic waves so as to generate ultrasonic images to be used for diagnoses. Further, the present invention relates to a data analysis and measurement apparatus for performing analysis of data obtained by such an ultrasonic diagnostic apparatus and/or measurement based on the data.

### Description of a Related Art

In an ultrasonic diagnostic apparatus to be used for medical purposes, an ultrasonic probe including plural ultrasonic transducers having functions of transmitting and receiving ultrasonic waves is typically used. An object to be inspected is scanned with an ultrasonic beam formed by synthesizing plural ultrasonic waves and ultrasonic echoes reflected within the object are received by using the ultrasonic probe, and thereby, image information on tissues of the object is obtained based on the intensity of the ultrasonic echoes.

As a related technology, Japanese Patent Application Publication JP-A-6-30930 discloses an ultrasonic diagnostic apparatus in which image quality is not deteriorated even when an image is enlarged at the time of cine-memory reproduction. The ultrasonic diagnostic apparatus includes an A/D converter that operates on a sampling clock at a faster rate than a sample rate for obtaining data necessary for normal image display, a cine-memory for storing a necessary amount of data for image enlargement, a data thinning circuit for thinning data according to an image enlargement factor, and a controller for outputting the sampling clock, outputting a cine-memory control signal to the cine-memory, outputting a thinning control signal to the thinning circuit, and outputting a DSC control signal to a DSC-so as to display enlarged images.

Further, Japanese Patent Application Publication JP-P2001-299745A discloses an ultrasonic diagnostic apparatus in which scaling of monochrome images, Doppler sample point depth shift, CFMBOX depth shift, and various image processing setting parameters can be varied even after freeze by directly storing in a cine-memory circuit an RF signal obtained based on phase-shifted detection signals from an ultrasonic receiving unit. According to the ultrasonic diagnostic apparatus, since various parameters can be varied even after freeze, reimaging is reduced and the operability is improved.

However, JP-A-6-30930 and JP-P2001-299745A never specifically disclose an ultrasonic diagnostic apparatus for performing data analysis or measurement by using ultrasonic diagnostic image data stored in a memory.

Furthermore, Japanese Patent Application Publication JP-P2005-118314A discloses an ultrasonic diagnostic apparatus capable of measuring an intima-media thickness (IMT) value of a carotid artery with high accuracy. The ultrasonic diagnostic apparatus includes an ultrasonic probe for transmitting ultrasonic waves into a body of an examinee, receiving ultrasonic waves reflected within the body, and converting the ultrasonic waves into a high-frequency electric signal, A/D converting means for converting the high-frequency electric signal into high-frequency digital data, high-frequency digital data storing means for storing the high-frequency digital data, image data converting means for converting the high-frequency digital data into digital data for image display, image display means for displaying an image based on the data for image display, and data analysis means for acquiring the high-frequency digital data from the high-frequency digital data storing means and performing predetermined analysis thereon.

According to the ultrasonic diagnostic apparatus, since the processing suitable for IMT value measurement is performed on the high-frequency digital data from which information has not been eliminated by logarithmic compression or the like, the respective data analyses may be correctly performed for any purpose. Further, a configuration provided with a cine-data memory for storing image data for display converted in the image data converting means in addition to the high-frequency data cine-memory is also proposed. In this case, the data for image display is loaded from the cine-data memory when a past image is displayed on a monitor, and data stored in the high-frequency data cine-memory is loaded and converted into image data for measurement when data analysis such as IMT value calculation or the like is performed.

However, in JP-P2005-118314A, the data for image display loaded from the cine-data memory in the moving image observation function is data after image processing has been performed thereon, and therefore, sufficient scaling factors or signal processing parameters for identifying the part for measurement cannot be set.

In accordance with the preamble of claim 1, US 6 322 505 B1 discloses an ultrasonic diagnostic apparatus. In the prior art apparatus only a single data path is provided between an array of the ultrasonic transducers and the display unit which would exclude the possibility to generate an image signal selectively on the actual data or the data stored in the cine-memory.

### SUMMARY OF THE INVENTION

The present invention has been achieved in view of the above-mentioned circumstances. A purpose of the present invention is to provide an ultrasonic diagnostic apparatus having a reproducing function at the same high frame rate as that at the time of ultrasonic imaging and capable of analysis or measurement with high accuracy in moving picture observation by reproduction. A further purpose of the present invention is to provide a data analysis and measurement apparatus for performing analysis of data obtained by such an ultrasonic diagnostic apparatus and/or measurement based on the data.

In order to accomplish the above-mentioned purposes, an ultrasonic diagnostic apparatus according to one aspect of the present invention includes the features of claim 1.

Further, a data analysis and measurement apparatus according to one aspect of the present invention is an apparatus connected via a network to an ultrasonic diagnostic apparatus according to the present invention and a data accumulation apparatus for accumulating compressed RF data with the information on frame rate, and the data analysis and measurement apparatus includes: a communication unit for receiving the compressed RF data with the information on frame rate from the data accumulation apparatus via a network; a data decompressing unit for decompressing the compressed RF data received by the communication unit; a cine-memory for storing the RF data decompressed by the data decompressing unit with the information on frame rate; a cine-memory reproducing unit for generating address information of the RF data in synchronization with a clock signal having a frequency controlled based on the information on frame rate stored in the cine-memory when the RF data is loaded from the cine-memory; a data analyzing and measuring unit for performing analysis and/or measurement on a selected region of interest based on the RF data loaded from the cine-memory; an image signal generating unit for generating an image signal based on the RF data loaded from the cine-memory; and a display unit for displaying an ultrasonic image based on the image signal generated by the image signal generating unit.

According to the present invention, moving images at the equally high frame rate to that at the time of ultrasonic imaging can be reproduced even after ultrasonic imaging by storing the RF data in the cine-memory, and therefore, ROI (region of interest) can be identified and analysis and/or measurement can be performed with high accuracy in moving image observation by cine-memory reproduction. Further, by compressing the RF data and accumulating the RF data in the data accumulation apparatus, the RF data can be centrally managed with examination information on the network, and the storage capacity of the ultrasonic diagnostic apparatus can be reduced to suppress the cost. In the case where the analysis and/or measurement is performed by using the RF data accumulated in the data accumulation apparatus, the data analysis and measurement apparatus without ultrasonic transmitting and receiving functions can be used.

### BRIEF DESCRIPTION OF THE DRAWINGS

Fig. 1 is a block diagram showing a configuration of an ultrasonic diagnostic apparatus according to the first embodiment of the present invention;
Figs. 2A and 2B show examples of button arrangement in a console of an input unit;
Fig. 3 is a block diagram showing a configuration of a cine-memory reproducing unit;
Figs. 4A and 4B show examples of a screen displayed on a touch panel in a cine-memory reproduction mode;
Fig. 5 is a block diagram showing a configuration of an ultrasonic diagnostic system including an ultrasonic diagnostic apparatus according to the second embodiment of the present invention;
Fig. 6 is a block diagram showing a configuration of an RF data compressing unit included in the ultrasonic diagnostic apparatus shown in Fig. 5;
Fig. 7 is a block diagram showing a configuration of a data analysis and measurement apparatus included in the ultrasonic diagnostic system shown in Fig. 5;
Fig. 8 is a flowchart showing an operation in an online diagnostic mode of the ultrasonic diagnostic system; and
Fig. 9 is a flowchart showing an operation in an offline diagnostic mode of the ultrasonic diagnostic system.

### DESCRIPTION OF THE PREFERRED EMBODIMENTS

Hereinafter, preferred embodiments of the present invention will be described in detail with reference to the drawings. The same reference numbers are assigned to the same component elements and the description thereof will be omitted.

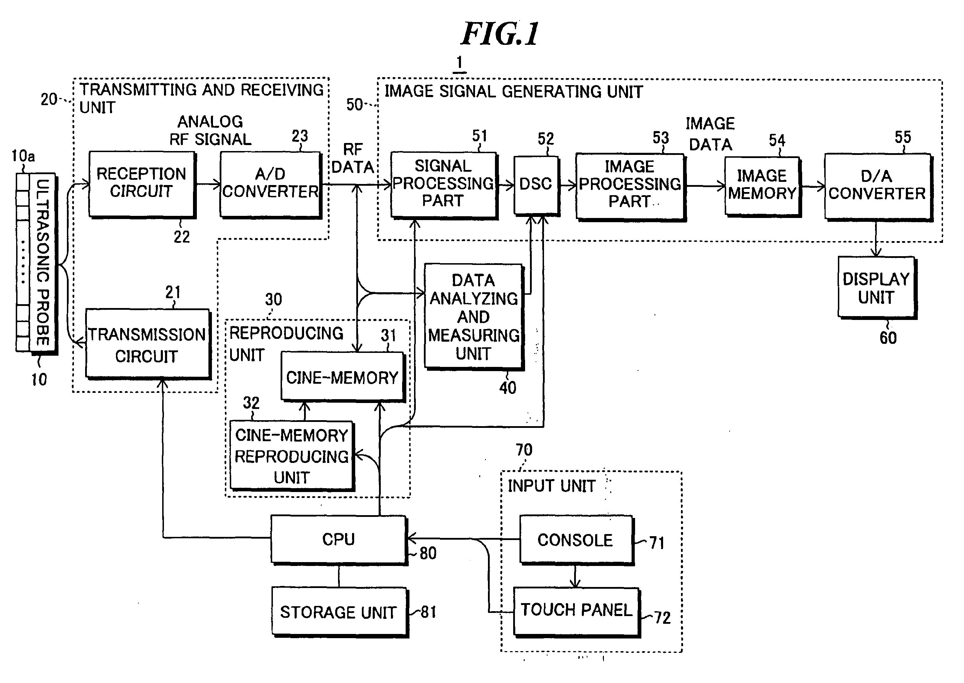

Fig. 1 is a block diagram showing a configuration of an ultrasonic diagnostic apparatus according to the first embodiment of the present invention. The ultrasonic diagnostic apparatus 1 includes an ultrasonic probe 10, a transmitting and receiving unit 20, a reproducing unit 30, a data analyzing and measuring unit 40, an image signal generating unit 50, a display unit 60, an input unit 70, a CPU 80, and a storage unit 81.

The ultrasonic probe 10 for use in contact with an object to be inspected includes plural ultrasonic transducers 10a that form a one-dimensional or two-dimensional transducer array. These ultrasonic transducers 10a transmit ultrasonic beams to the objectbased on applied drive signals, and receive ultrasonic echoes reflected from the object to output detection signals.

Each ultrasonic transducer is configured of a vibrator in which electrodes are formed on both ends of a material havingapiezoelectricproperty (piezoelectricmaterial) such as a piezoelectric ceramic represented by PZT (Pb (lead) zirconate titanate), a polymeric piezoelectric element represented by PVDF (polyvinylidene difluoride), or the like. When a voltage is applied to the electrodes of the vibrator by transmitting pulse or continuous wave electric signals, the piezoelectric material expands and contracts. By the expansion and contraction, pulse or continuous wave ultrasonic waves are generated from the respective vibrators, and an ultrasonic beam is formed by synthesizing these ultrasonic waves. Further, the respective vibrators expand and contract by receiving propagating ultrasonic waves and generate electric signals. These electric signals are outputted as detection signals of ultrasonic waves.

Alternatively, as the ultrasonic transducers, plural kinds of elements of different ultrasonic conversion types may be used. For example, the above-mentioned vibrators are used as elements for transmitting ultrasonic waves and photo-detection type ultrasonic transducers are used as elements for receiving ultrasonic waves. The photo-detection type ultrasonic transducer is for detecting ultrasonic waves by converting ultrasonic signals into optical signals, and configured of a Fabry-Perot resonator or fiber Bragg grating, for example.

The storage unit 81 is configured of a hard disk or memory, and stores software (program) for allowing the CPU 80 to execute the control of the respective parts of the ultrasonic diagnostic apparatus 1. Therefore, the CPU 80 and the software correspond to a control unit of the ultrasonic diagnostic apparatus 1. The control unit has a scanning control function for sequentially setting the transmission directions of ultrasonic beams and reception directions of ultrasonic echoes, a transmission control function for selecting a transmission delay pattern according to the set transmission direction, and a reception control function for selecting a reception delay pattern according to the set transmission direction.

Here, the transmission delay pattern refers to pattern data of delay time to be provided to the drive signals so as to form an ultrasonic beam in a desired direction with the ultrasonic waves transmitted by the plural ultrasonic transducers 10a, and the reception delay pattern refers to pattern data of delay time to be provided to the detection signals so as to extract ultrasonic echoes from the desired direction with the ultrasonic waves received by the plural ultrasonic transducers 10a. Plural transmission delay patterns and reception delay patterns are stored in the storing means such as a memory or the like.

The transmitting and receiving unit 20 includes a transmission circuit 21, a reception circuit 22, and an A/D converter 23. Further, the reproducing unit 30 includes a cine-memory 31 and a cine-memory reproducing unit 32. Furthermore, the image signal generating unit 50 includes a signal processing part 51, a DSC (Digital Scan Converter) 52, an image processing part 53, an image memory 54, and a D/A converter 55.

The transmission circuit 21 generates drive signals to be respectively applied to the plural ultrasonic transducers 10a, and is able to provide respective delay times to the drive signals based on the transmission delay pattern selected by the CPU 80. The transmission circuit 21 may adjust the delay amounts of the drive signals and supply the drive signals to the ultrasonic probe 10 such that the ultrasonic waves transmitted from the plural ultrasonic transducers 10a form an ultrasonic beam, or may supply drive signals to the ultrasonic probe 10 such that the ultrasonic waves transmitted at once from the plural ultrasonic transducers 10a reach the entire imaging region of the object.

The reception circuit 22 amplifies the detection signals respectively outputtedfrom the plural ultrasonic transducers 10a, and provides respective delay times to the detection signals based on the reception delay pattern selected by the CPU 80 and adding those detection signals one another to perform reception focus processing. Due to the reception focus processing, sound ray signals (hereinafter, also referred to as "RF signals") are formed, in which the focal point of the ultrasonic echoes is narrowed. The A/D converter 23 converts the analog RF signals into digital RF signals (hereinafter, also referred to as "RF data").

The RF data outputted f rom the A/D converter 2 3 is inputted to the cine-memory 31 and the signal processing part 51. The cine-memory 31 sequentially stores the RF data inputted from the A/D converter 23. Further, the cine-memory 31 inputs parameters such as depths of reflection positions of ultrasonic waves, density of scan lines, and viewing width as information on frame rate from the CPU 80 and stores it in association with the RF data.

The signal processing part 51 corrects attenuation owing to distance on the RF data according to the depths of the reflection positions of ultrasonic waves in STC (Sensitivity Time gain Control), and then, performs envelope detection processing thereon to generate B-mode image data.

In the above-mentioned example, the detection signals subjected to reception focus processing in the reception circuit 22 are used as RF signals, however, detection signals that have not been subjected to reception focus processing may be used as RF signals. In this case, detection signals respectively outputted from the plural ultrasonic transducers 10a are amplified in the reception circuit 22, the amplified detection signals, i.e., the RF signals are A/D-converted by the A/D converter 23 to generate RF data. The RF data is supplied to the signal processing part 51 and stored in the cine-memory 31. The reception focus processing is digitally performed in the signal processing part 51.

Thus, in the cine-memory reproduction mode, observation of moving images focused on a position different from that in the live mode can be made. However, in the case where the detection signals that have not been subjected to reception focus processing are used as RF signals, since the amount of data increases, in order to prevent the rise in costs, it is desirable to adopt an imaging method such as an aperture synthesis method of performing wavefront synthesis by which spatial information can be acquired at the smaller number of transmission times.

In the case where the aperture synthesis method is adopted, the transmission circuit 21 supplies drive signals to the ultrasonic probe 10 such that ultrasonic waves transmitted from the plural ultrasonic transducers 10a at once can reach the entire imaging region of the object. Further, in the transmitting and receiving unit 20, detection signals obtained by the plural ultrasonic transducers 10a receiving ultrasonic waves at plural different times are amplified by the reception circuit 22, and the amplified detection signals are sequentially A/D converted in the A/D converter 23 to generate time-series RF data. The RF data is supplied to the signal processing part 51 and stored in the cine-memory 31.

Further, in the image signal generating unit 50, the time-series RF data is once stored in a memory for aperture synthesis . Furthermore, one suitable set of time-series data is selected according to the positions of the plural ultrasonic transducers 10a from the time-series RF data stored in the memory for aperture synthesis, values of the respective time-series data are added to perform wavefront synthesis, and thereby, sound ray data in which a focal point is formed on one point within the imaging region is obtained. The same processing is performed on the respective points within the imaging region.

Since the B-mode image data generated by the signal processing part 51 is obtained by the scan system different from the normal scan system for television signals, the DSC 52 converts (raster-converts) the data into normal image data. The image processing part 53 performs various kinds of necessary image processing such as gradation processing on the image data inputted from the DSC 52.

The image memory 54 stores image data inputted from the image processing part 53. The D/A converter 55 converts the image data loaded from the image memory 54 into analog image signals and outputs the signals to the display unit 60. Thereby, in the display unit 60, ultrasonic diagnostic images imaged by the ultrasonic probe 10 are displayed.

The input unit 70 includes a console 71 to be used by a user to operate the ultrasonic diagnostic apparatus 1, and a touch panel 72 as user interface means to be used to display an operation screen and input instructions when reproduction of the RF data stored in the cine-memory 31 is performed (cine-memory reproduction) . As the user interface means , the display unit 60 for displaying an ultrasonic diagnostic image and input means such as a mouse may be used other than the touch panel 72.

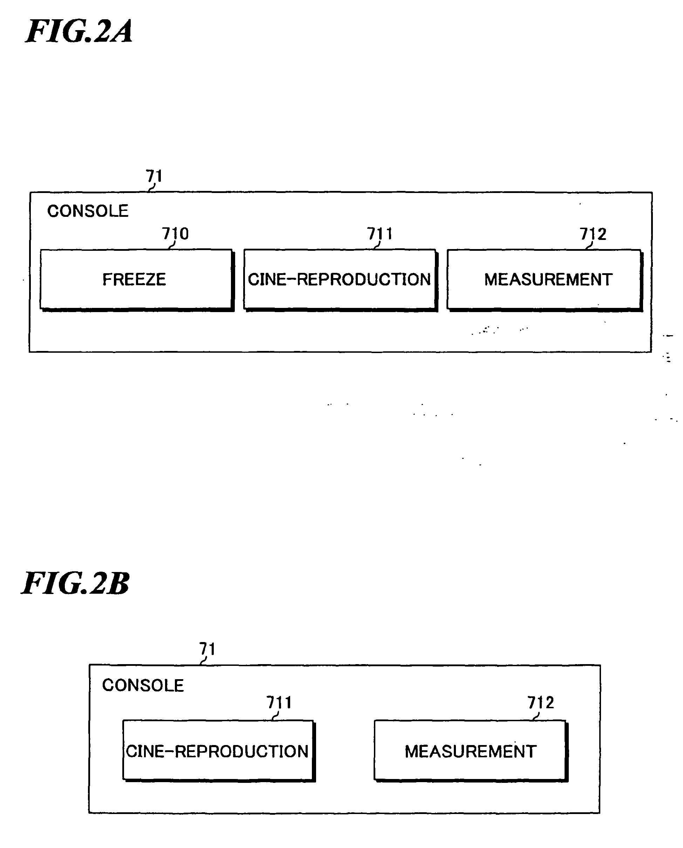

Fig. 2A shows one example of button arrangement in the console of the input unit. In the embodiment, the console 71 is provided with a freeze button 710 for instructing the switching between the live reproduction function and the freeze function, a cine-reproduction button 711 for instructing cine-memory reproduction, and a measurement button 712 for instructing analysis and/or measurement using the RF data. By pushing down the freeze button 710, cine-reproduction button 711, and measurement button 712, their instructions are notified to the CPU 80. Fig. 2B shows another example of the button arrangement in the console of the input unit, and this will be described later.

In the case where an ultrasonic diagnostic image is displayed by performing cine-memory reproduction, it is necessary to load the RF data stored in the cine-memory 31 at an appropriate frame rate determined by parameters such as the depth of reflection position of ultrasonic wave, the density of scan lines, and viewing width. In the embodiment, the frame rate information to be used for determining the frame rate at the time of reproduction is stored in the cine-memory 31 with the RF data.

The CPU 80 outputs a control signal for controlling the frequency of a reproduction clock signal to the cine-memory reproducing unit 32 based on the frame rate information loaded from the cine-memory 31. The cine-memory reproducing unit 32 generates the reproduction clock signal having the frequency according to the control signal inputted from the CPU 80. Thus, based on the frame rate information, the frequency of the reproduction clock signal, and further, the frame rate at the time of reproduction are determined. The cine-memory reproducing unit 32 generates address information of the RF data to be loaded from the cine-memory 31 in synchronization with the reproduction clock signal and outputs the address information to the cine-memory 31.

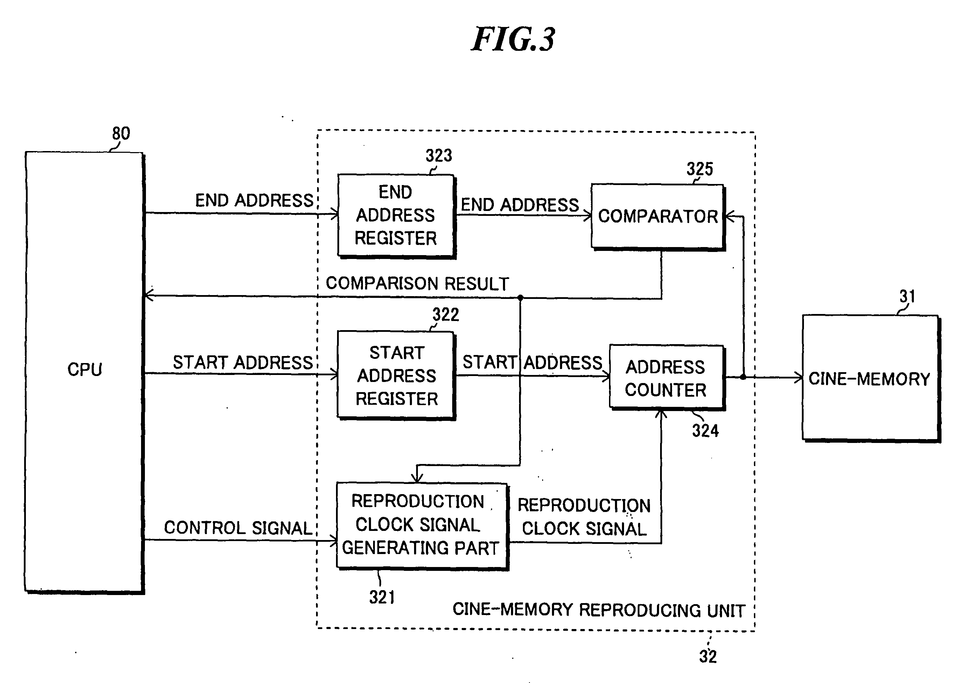

Fig. 3 is a block diagram showing a configuration of the cine-memory reproducing unit. The cine-memory reproducing unit 32 includes a reproduction clock signal generating part 321, a start address register 322, an end address register 323, an address counter 324, and a comparator 325. The start address register 322 inputs the start address of the RF data from the CPU 80 and stores it. The end address register 323 inputs the end address of the RF data from the CPU 80 and stores it.

In the case where the ultrasonic diagnostic image is displayed by performing cine-memory reproduction, the reproduction clock signal generating part 321 inputs the control signal generated by the CPU 80 based on the frame rate information, generates the reproduction clock signal having the frequency adapted to the frame rate of the RF data to be loaded according to the control signal, and supplies the reproduction clock signal to the address counter 324.

The address counter 324 has a counter function of inputting the start address from the start address register 322 and incrementing the start address in synchronization with the reproduction clock signal. The address obtained by the address counter 324 is sequentially inputted to the cine-memory 31 and the comparator 325. In the cine-memory reproduction, the RF data loaded from the cine-memory 31 is supplied to the image signal generating unit 50, and an image signal is generated.

The comparator 325 compares the address inputted from the address counter 324 and the end address inputted from the end address register 323 to detect the end of the cine-memory reproduction, and outputs the detection result to the CPU 80 and the reproduction clock signal generating part 321. When the end of the cine-memory reproduction is detected, the reproduction clock signal generating part 321 stops the generation of the reproduction clock signal.

Referring to Fig. 1 again, the data analyzing and measuring unit 40 has a function of performing analysis and/or measurement designated by the user of the ultrasonic diagnostic apparatus 1. In the embodiment, the data analyzing and measuring unit 40 is able to perform tissue part distortion analysis (hardness diagnosis), bloodstream measurement, tissue part motion measurement, or IMT (Intima-Media Thickness) value measurement. When the user pushes down the measurement button 712 of the console 71, the data analyzing and measuring unit 40 performs desired analysis and/or measurementbasedon the RF data inputted from the A/D converter 23 or the cine-memory 31. The analysis result and/or measurement result is outputted to the DSC 52 of the image signal generating unit 50, and inserted to the ultrasonic tomographic image by the DSC 52.

The CPU 80 controls the respective functional blocks according to the operation mode of the ultrasonic diagnostic apparatus 1. For example, when the user pushed down the freeze button 710 of the console 71, the CPU 80 controls the transmitting and receiving unit to switch between the live state and the freeze state. Further, the data analyzing and measuring unit 40, signal processing part 51, DSC 52, and image processing part 53 may be configured of the CPU 80 and software (program).

Here, the operation of the ultrasonic diagnostic apparatus 1 in the live mode will be explained with reference to Figs. 1 and 2.

The live mode refers to a mode in which display and analysis and/or measurement of ultrasonic images is performed based on RF data obtained by bringing the ultrasonic probe in contact with the object and transmitting and receiving ultrasonic waves. In the live mode, the digital RF signal outputted from the A/D converter 23 is processed in the signal processing part 51 and B-mode image data is generated. Simultaneously, the cine-memory 31 sequentially stores the RF data inputted from the A/D converter 23.

The DSC 52 converts the B-mode image data into image data for normal scan system. The image processing part 53 performs various kinds of image processing such as gradation processing on the image data inputted from the DSC 52. The image memory 54 stores the image data converted by the DSC 52. The D/A converter 55 converts the image data loaded form the image memory 54 into an analog image signal and outputs it to the display unit 60. Thereby, in the display unit 60, the ultrasonic diagnostic image imaged by the ultrasonic probe 10 is displayed.

The user is able to acquire a desired still image by pushing down the freeze button 710 when observing the moving images in the live mode, and specify the ROI. Further, the user is able to allow the data analyzing and measuring unit 40 to perform desired analysis and/or measurement based on the RF data that has not been subjected to image processing by pushing down the measurement button 712.

Next, the operation of the ultrasonic diagnostic apparatus 1 in the cine-memory reproduction mode will be explained with reference to Figs. 1-4B.

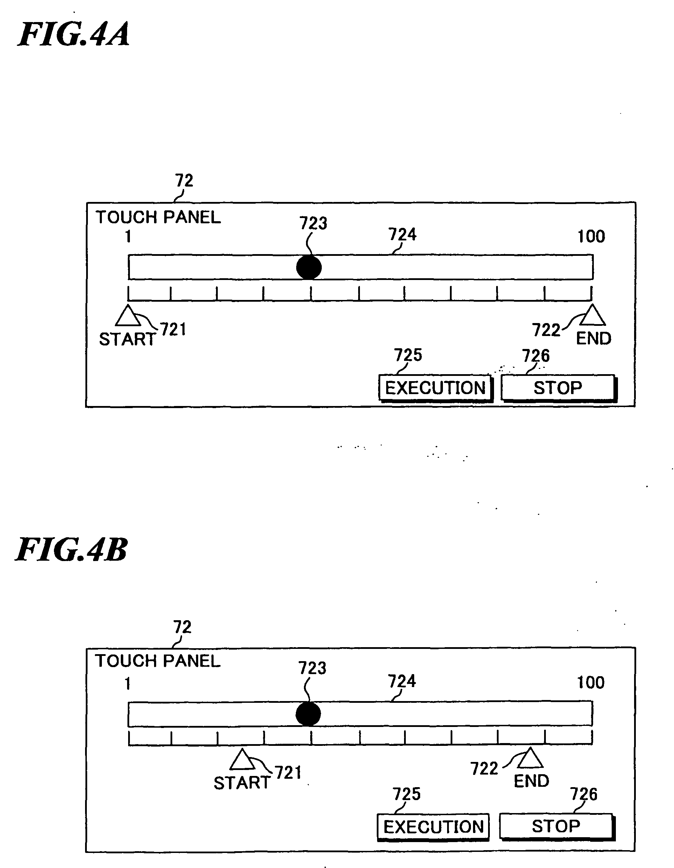

The cine-memory reproduction mode refers to a mode in which display and analysis and/or measurement of ultrasonic images is performed based on RF data stored in the cine-memory 31. In the cine-memory reproduction mode, imaging of the object is not necessary. When the user pushes down the cine-memory reproduction button 711, a screen shown in Fig. 4A is displayed on the touch panel 72.

Fig. 4A shows an example of the screen displayed on the touch panel in the cine-memory reproduction mode. When the reproduction time of RF data stored in the cine-memory 31 is very long, it is desirable that the user can reproduce only the desired time zone. On the screen of the touch panel shown in Fig. 4A, reproduction in the desired time zone can be designated by a start pointer 721 and an end pointer 722 on a reproduction bar 724. The start pointer 721 and the end pointer 722 are horizontally slidable on the reproduction bar 724, and therefore, the start pointer 721 and the end pointer 722 can be designated as shown in Fig. 4B, for example. In Figs. 4A and 4B, a reproduction pointer 723 indicates the point that is currently reproduced, and moves from left to right on the reproduction bar 724 in real time.

After the user designates the start pointer 721 and the end pointer 722, by pushing down an execution button 725 on the touch panel 72, the RF data in the designated region is loaded from the cine-memory 31 and reproduction of the ultrasonic tomographic image (cine-memory reproduction) is performed on the loaded RF data.

When the user pushes down the execution button 725 on the touch panel 72, the CPU 80 stores as the start address the address of the RF data corresponding to the start pointer 721 in the start address register 322 of the cine-memory reproducing unit 32. Similarly, the CPU 80 stores as the end address the address of the RF data corresponding to the end pointer 722 in the end address register 323 of the cine-memory reproducing unit 32.

Further, the CPU 80 generates the control signal based on the frame rate information loaded from the cine-memory 31 and outputs the control signal to the reproduction clock signal generating part 321 of the cine-memory reproducing unit 32. The reproduction clock signal generating part 321 generates a reproduction clock signal having a frequency according to the inputted control signal and supplies it to the address counter 324.

The address counter 324 starts operation when supplied with the reproduction clock signal from the reproduction clock signal generating part 321, and acquires the start address from the start address register 322. The address counter 324 increments the count value in synchronization with the clock signal by using the start address as the initial value of the count value. The count value obtained by the address counter 324 is outputted to the cine-memory 31 and the comparator 325 as address information.

When the address information is inputted from the address counter 324 to the cine-memory 31, the RF data corresponding to the address information stored in the cine-memory 31 is loaded and outputted to the signal processing part 51. Here, since the generation of the address information is performed in synchronization with the reproduction clock signal generated in the reproduction clock signal generating part 321, the loading of the RF data from the cine-memory 31 is performed at the same frame rate as that in the live mode. That is, in the cine-memory reproduction mode, reproduction of the ultrasonic diagnostic image is performed at the same frame rate as that in the live mode.

In the cine-memory reproduction mode, on the touch panel 72, the reproduction pointer 723 moves from the position of the designated start pointer 721 to the position of the designated end pointer 722. Alternatively, the user is able to stop the cine-memory reproduction by pushing down a stop button 726 on the touch panel 72 during the cine-memory reproduction. Thus, desired analysis and/or measurement can be performed by pushing down the stop button 726 to acquire a desired still image and identify ROI.

In the case where analysis and/or measurement using RF data is performed, the user operates the input unit 70 to designate desired analysis and/or measurement, and then, pushes down the measurement button 712 of the input unit 70. The CPU 80 receives the signal from the input unit 70 and instructs the designated analysis and/or measurement to the data analyzing and measuring unit 40. The data analyzing and measuring unit 40 performs the designated data analysis and/or measurement based on the RF data loaded from the cine-memory 31. The data analysis result and/or measurement result obtained in the data analyzing and measuring unit 40 is outputted to the display unit 60 via the DSC 52 and so on, and displayed on the display unit 60.

The comparator 325 compares the end address stored in the end address register 323 and the address inputted from the address counter 324. When the comparator 325 detects that the address information inputted from the address counter 324 reaches the end address, it notifies the CPU 80 and the reproduction clock signal generating part 321 of the detection result.

Thereby, when the reproduction pointer 723 moves to the end pointer 722 on the touch panel 72, the CPU 80 ends the cine-memory reproduction. Here, cine-loop reproduction for continuously loop-reproducing the ultrasonic diagnostic image may be performed by the CPU 80 setting-the start address in the start address register 322 again.

Generally, in hardness diagnosis, moving image observation is important to recognize a lesion part. For example, when a hardened tissue part is diagnosed in the beat of a heart or the like, the hardened tissue part is slower in motion than other normal tissue parts. In this case, moving image observation as that in the live is effective for diagnosis.

In the embodiment, since the moving image reproduction at the same frame rate as that in the live mode can be realized in the cine-memory reproduction, the same moving image observation as that in the live mode can be performed even when the object is notpresent. Further, since the cine-memory reproduction is performed by using RF data that has not been subjected to image processing, desired data analysis and/or measurement can be performed by identifying ROI while changing parameters such as the scaling factor, gain, contrast, and STC during the cine-memory reproduction. Here, since the timing with which the ultrasonic probe 10 transmits or receives ultrasonic waves differs according to the depth of the diagnostic location (reflection positions of the ultrasonic waves), the scan system of the ultrasonic probe 10, or the like, the motion of the object can be faithfully reproduced by reproducing the RF data stored in the cine-memory 31 at an appropriate frame rate.

Next, the second embodiment of the present invention will be explained.

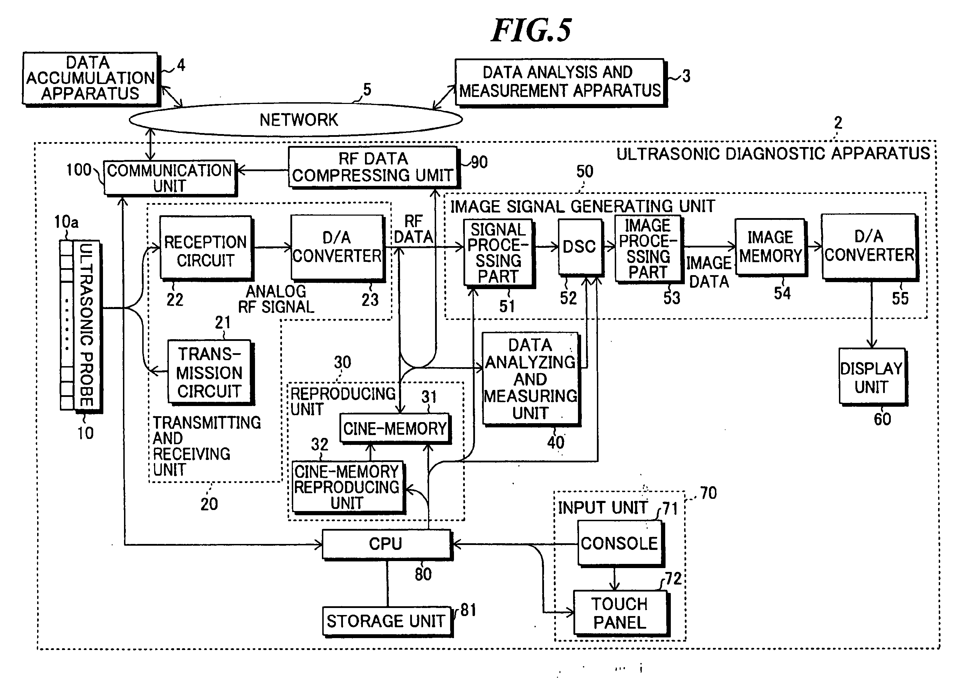

Fig. 5 is a block diagram showing a configuration of an ultrasonic diagnostic system including an ultrasonic diagnostic apparatus according to the second embodiment of the present invention. The ultrasonic diagnostic apparatus according to the second embodiment has an RF data compressing unit 90 and a communication unit 100 in addition to the configuration of the ultrasonic diagnostic apparatus according to the first embodiment shown in Fig. 1. The communication unit 100 is connected to a data analysis and measurement apparatus 3 and a data accumulation apparatus 4 via a network 5, and makes communication between these apparatuses and itself.

The data accumulation apparatus 4 manages patient information such as patient ID and examination information such as examination ID, and an HDD (Hard Disc Drive), a file server, or the like may be used as the data accumulation apparatus 4. As the network 5, for example, LAN (Local Area Network) such as Ethernet (registered trade mark), WAN (Wide Area Network), Internet, or wireless LAN for transmitting and receiving data with wireless communication may be used.

In Fig. 5, the RF data outputted from the A/D converter 23 or the cine-memory 31 is inputted to the RF data compressing unit 90. The RF data compressing unit 90 performs data compression processing on the RF data. The RF data that has subjected to the compression processing by the RF data compressing unit 90 and the frame rate information outputted from the CPU 80 are transmitted from the communication unit 100 via the network 5 to the data accumulation apparatus 4. The data accumulation apparatus 4 accumulates the received RF data and frame rate information.

The RF data inputted to the RF data compressing unit 90 may be data obtained by A/D-converting RF signals generated by amplifying detection signals respectively outputted from the plural ultrasonic transducers 10a and adjusting the delay amounts of the detection signals and adding them one another, or may be data obtained by A/D-converting RF signals generated by amplifying detection signals respectively outputted from the plural ultrasonic transducers 10a.

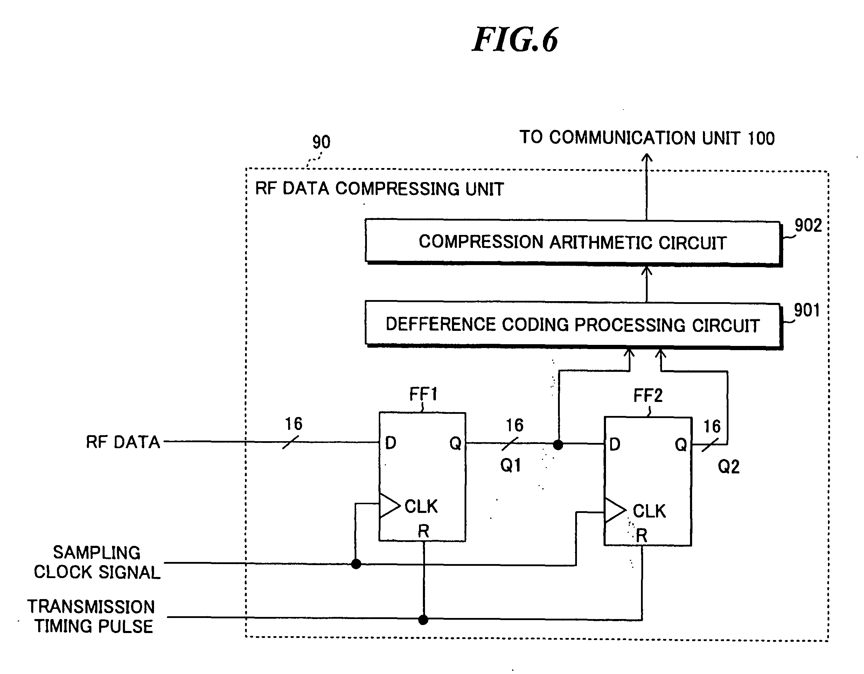

Fig. 6 is a block diagram showing a configuration of the RF data compressing unit included in the ultrasonic diagnostic apparatus shown in Fig. 5 . The RF data compressing unit 90 includes D-flip-flops FF1 and FF2 , a difference coding processing circuit 901, and a compression arithmetic circuit 902. In the embodiment, RF data is 16-bit data.

RF data is inputted to the input terminal D of the flip-flop FF1. The output signal Q1 of the flip-flop FF1 is inputted to the data input terminal D of the flip-flop FF2. Further, the output signal Q1 of the flip-flop FF1 and the output signal Q2 of the flip-flop FF2 are inputted to the difference coding processing circuit 901 and difference coding processing is performed thereon. The data outputted from the difference coding processing circuit 901 is inputted to the compression arithmetic circuit 902, data compression is performed thereon in the compression arithmetic circuit 902, and then, the compressed data is outputted to the communication unit 100.

Further, sampling clock signals having a frequency four to eight times the frequency of the ultrasonic waves (the frequency of the drive signals or detection signals) are inputted to clock signal input terminals CLK of the flip-flops FF1 and FF2 . The frequency of the sampling clock signals is determined according to the design condition because it has influence on the difference coding of the RF data.

Furthermore, transmission timing pulses are inputted to reset terminals R of the flip-flops FF1 and FF2 . When data transmission is not performed, the transmission timingpulses are set to reset the flip-flops FF1 and FF2. On the other hand, when data transmission is performed, the transmission timing pulses are set to operate the flip-flops FF1 and FF2, and output data Q1 and Q2 of the flip-flops FF1 and FF2 are outputted to the difference coding processing circuit 901. The difference coding processing circuit 901 performs difference coding processing by using the output data Q1 and Q2 of the flip-flops FF1 and FF2.

In the difference coding processing, data on the Nth sampling point and data on the (N-l)th sampling point are extracted by using the D-flip-flop and a difference is obtained by subtracting one from the other, and thereby, statistical deviation is caused in the data to make the value of data smaller.

For example, the compression arithmetic circuit 902 calculates a run length and performs Huffman coding processing on the data that has been subjected to difference coding processing, and thereby, compresses the data. Alternatively, the compressed data may be formed by shortening the bit lengths of the data that has been subjected to difference coding processing and packing the plural data in the compression arithmetic circuit 902.

In the ultrasonic diagnostic system, the mode, in which diagnoses are made by acquiring ultrasonic images by using the ultrasonic diagnostic apparatus 2 connected to the data analysis and measurement apparatus 3 via the network 5, is referred to as "online diagnostic mode" . On the other hand, the mode, in which diagnoses are made by using the data analysis and measurement apparatus 3 under a condition that the ultrasonic diagnostic apparatus 2 is not connected to the network 5 or power of the ultrasonic diagnostic apparatus 2 is not on, is referred to as "offline diagnostic mode".

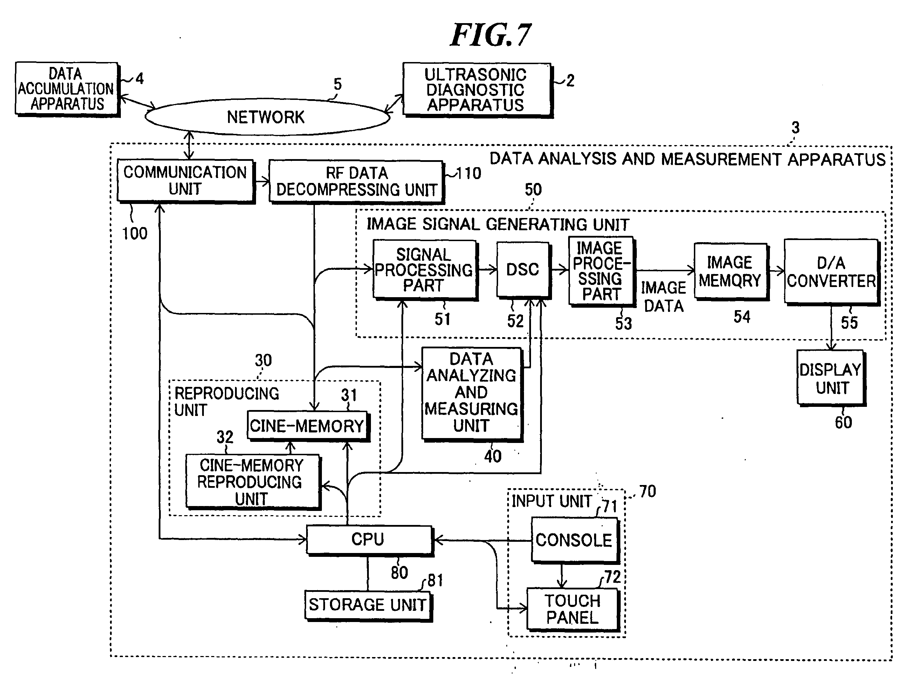

Fig. 7 is a block diagram showing a configuration of the data analysis and measurement apparatus included in the ultrasonic diagnostic system shown in Fig. 5. The data analysis and measurement apparatus 3 includes a reproducing unit 30, a data analyzing and measuring unit 40, an image signal generating unit 50, a display unit 60, an input unit 70, a CPU 80, a storage unit 81, a communication unit 100, and an RF data decompressing unit 110 . The data analysis and measurement apparatus 3 may be configured by employing a workstation for image processing or personal computer.

The communication unit 100 receives the compressed RF data with the frame rate information from the data accumulation apparatus 4 via the network, outputs the compressed RF data to the RF data decompressing unit 110, and outputs the frame rate information to the cine-memory 31.

The RF data decompressing unit 110 performs data decompression processing, which is opposite to the data compression processing in the RF data compressing unit 90 of the ultrasonic diagnostic apparatus 2, on the compressed RF data inputted from the communication unit 100. The RF data that has been subjected to data decompression processing is outputted to the cine-memory 31, and stored with the frame rate information in the cine-memory 31. Cine-memory reproduction can be performed based on the RF data and frame rate information stored in the cine-memory 31.

Next, the input unit 70 of the data analysis and measurement apparatus 3 will be explained.

Fig. 2B shows an example of button arrangement on a console of the input unit. In the embodiment, a cine-reproduction button 711 for instructing cine-memory reproduction and a measurement button 712 for instructing analysis and/or measurement using RF data are provided on the console 71. When the cine-reproduction button 711 or the measurement button 712 is pushed down, its instruction is notified to the CPU 80. In the data analysis and measurement apparatus 3, the freeze button 710 as shown in Fig. 2A is not necessary because the live-mode diagnosis using the ultrasonic probe 10 is notmade. In the data analysis and measurement apparatus 3, when the cine-reproduction button 711 on the console 71 is pushed down, a screen shown in Fig. 4A is displayed on the touch panel provided in the input unit 70. The operation of the input unit is the same as that described in the first embodiment.

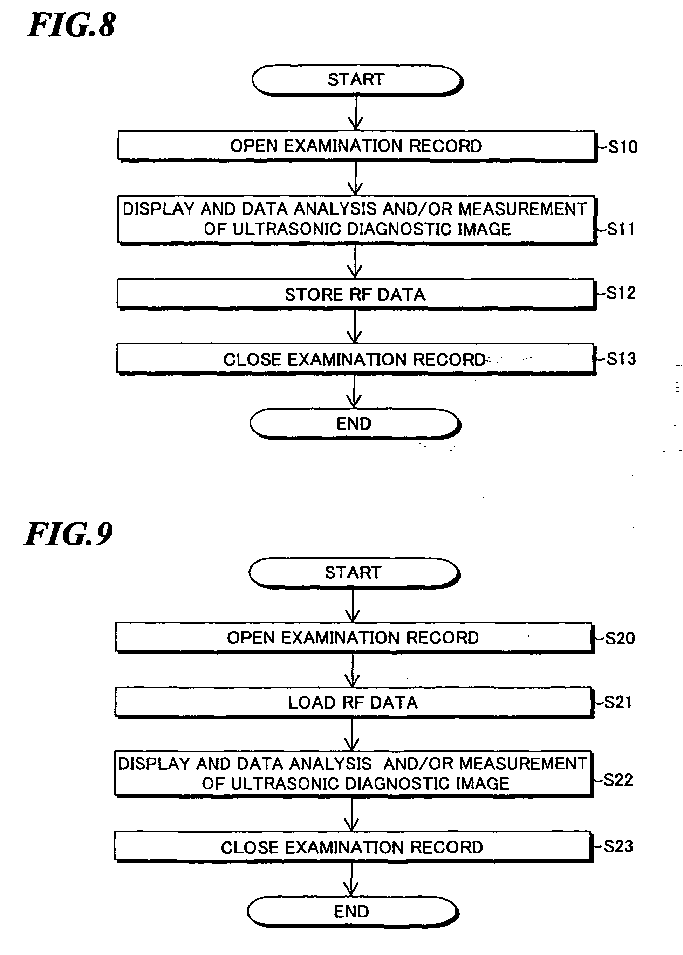

Here, the operation in the online diagnostic mode of the ultrasonic diagnostic system shown in Fig. 5 will be explained with reference to Fig. 8. Fig. 8 is a flowchart showing the operation in the online diagnostic mode of the ultrasonic diagnostic system.

When the online diagnosis is started, first, at step S10, patient information and examination information are transmitted from the ultrasonic diagnostic apparatus 2 via the network 5 to the data accumulation apparatus 4 according to the user' s operation at the ultrasonic diagnostic apparatus 2 , and a corresponding examination record of the examination records accumulated in the data accumulation apparatus 4 is opened.

At step S11, ultrasonic imaging in the live mode is performed by using the ultrasonic diagnostic apparatus 2. At this time, the ultrasonic diagnostic images of the object are displayed on the display unit and the generated RF data is stored in the cine-memory 31.

The user identifies ROI and instructs desired data analysis and/or measurement based on the ultrasonic diagnostic images obtained in the live mode or cine-memory reproduction mode. When instructing data analysis and/or measurement, the user pushes down the measurement button 712 (Fig. 2A) on the console 71. When the measurement button 712 is pushed down, the data analyzing and measuring unit 40 performs the designated data analysis and/or measurement based on the RF data outputted from the A/D converter 23 or cine-memory 31.

At step S12, the RF data outputted from the A/D converter 23 or cine-memory 31 is data-compressed in the RF data compressing unit 90, transmitted with the frame rate information from the ultrasonic diagnostic apparatus 2 via the network 5 to the data accumulation apparatus 4, and stored in the data accumulation apparatus 4. Thereby, the RF data can be centrally managed with the patient information and examination information and the diagnostic results on the network 5, and the storage capacity for accumulating the data in the ultrasonic diagnostic apparatus 2 can be reduced for suppressing costs.

At step S13, when the user operates to end examination, information representing the examination end is transmitted from the ultrasonic diagnostic apparatus 2 via the network 5 to the data accumulation apparatus 4, and the examination record stored in the data accumulation apparatus 4 is closed.

Next, the operation in the offline diagnostic mode of the ultrasonic diagnostic system shown in Fig. 5 will be explained with reference to Figs. 7 and 9. Fig. 9 is a flowchart showing the operation in the offline diagnostic mode of the ultrasonic diagnostic system.

When the offline diagnosis is started, first, at step S20, patient information and examination information are transmitted from the ultrasonic diagnostic apparatus 2 via the network 5 to the data accumulation apparatus 4 according to the user' s operation at the data analysis and measurement apparatus 3, and a corresponding examination record of the examination records accumulated in the data accumulation apparatus 4 is opened.

At step S21, the data analysis and measurement apparatus 3 loads the compressed RF data included in the corresponding examination record with the frame rate information from the data accumulation apparatus 4 via the network 5. This RF data is the RF data transmitted from the ultrasonic diagnostic apparatus 2 to the data accumulation apparatus 4 in the online mode. When the data analysis and measurement apparatus 3 acquires the RF data and the frame rate information from the data accumulation apparatus 4, the RF data decompressing unit 110 decompresses the RF data by performing decompression processing on the compressed RF data.

At step S22, the decompressed RF data is stored in the cine-memory 31 with the frame rate information, and the same processing as that in the ultrasonic diagnostic apparatus 2 in the online mode is performed thereon. That is, the RF data is loaded from the cine-memory 31 according to the operation of the console 71 by the user, processed in the signal processing part 51, the DSC 52, the image processing part 53, the image memory 54, and the D/A converter 55, and thereby, ultrasonic diagnostic images are displayed on the display unit 60.

The user identifies ROI and instructs desired data analysis and/or measurement based on the ultrasonic diagnostic images obtained in the cine-memory reproduction mode. When instructing data analysis and/or measurement, the user pushes down the measurement button 712 (Fig. 2B) on the console 71. When the measurement button 712 is pushed down, the data analyzing and measuring unit 40 loads the RF data from the cine-memory 31 and performs the designated data analysis and/or measurement. Since the RF data loaded from the cine-memory 31 has not been subjected to image display processing, it is preferable for use in data analysis and/or measurement of IMT value measurement or the like. Therefore, diagnoses with high accuracy can be realized.

At step S23, when the user operates to end examination, information representing the examination end is transmitted from the ultrasonic diagnostic apparatus 2 via the network 5 to the data accumulation apparatus 4, and the examination record stored in the data accumulation apparatus 4 are closed.

## Claims

1. An ultrasonic diagnostic apparatus comprising:
an ultrasonic probe (10) including plural ultrasonic transducers (10a) for transmitting ultrasonic waves to an object to be inspected according to drive signals and receiving ultrasonic echoes reflected from the object to output detection signals;
a transmitting and receiving unit (20) for supplying the drive signals to said ultrasonic probe (10), generating RF signals based on the detection signals, and A/D-converting the RF signals to generate RF data;
a cine-memory (31) for storing the RF data generated by said transmitting and receiving unit (20) with information on frame rate;
a cine-memory reproducing unit (32);
a data analyzing and measuring unit (40) for performing analysis and/or measurement on a selected region of interest based on RF data;
an image signal generating unit (50) for generating an image signal; and
a display unit (60) for displaying an ultrasonic image based on the image signal generated by said image signal generating unit (50), **characterized in that** said cine-memory reproducing unit (32) is adapted to generate address information of the RF data in synchronization with a clock signal having a frequency controlled based on the information on frame rate stored in said cine-memory (31) when the RF data is loaded from said cine-memory (31), that said data analysing and measuring unit (40) is adapted to perform analysis and/or measurement selectively on either the RF data generated by said transmitting and receiving unit (20) or the RF data loaded from said cine-memory (31), and said image signal generating unit (50) is adapted to generate an image signal selectively based on the RF data generated by said transmitting and receiving unit (20) or the RF data loaded from said cine-memory (31).

2. An ultrasonic diagnostic apparatus according to claim 1, wherein said transmitting and receiving unit (20) adjusts delay amounts of the drive signals and supplies the drive signals to said ultrasonic probe (10) such that ultrasonic waves transmitted from said plural ultrasonic transducers (10a) form an ultrasonic beam.

3. An ultrasonic diagnostic apparatus according to claim 1, wherein said transmitting and receiving unit (20) supplies the drive signals to said ultrasonic probe (10) such that ultrasonic waves transmitted at once from said plural ultrasonic transducers (10a) reach an entire imaging region of the object.

4. An ultrasonic diagnostic apparatus according to claim 1, wherein said transmitting and receiving unit (20) generates the RF signals by amplifying the detection signals outputted from said plural ultrasonic transducers (10a), adjusting delay amounts of the detection signals, and adding the detection signals one another.

5. An ultrasonic diagnostic apparatus according to claim 1, wherein said transmitting and receiving unit (20) generates the RF signals by amplifying the detection signals outputted from said plural ultrasonic transducers (10a).

6. An ultrasonic diagnostic apparatus according to claim 3, wherein said transmitting and receiving unit (20) generates the RF signals by performing wavefront synthesis based on the detection signals obtained by receiving the ultrasonic echoes at plural different times.

7. An ultrasonic diagnostic apparatus according to any one of claims 1-6, further comprising:
a control unit (80) for controlling the frequency of said clock signal based on the information on frame rate stored in said cine-memory (31).

8. An ultrasonic diagnostic apparatus according to any one of claims 1-6, further comprising:
an input unit (70) to be used for setting a desired reproduction start point and/or a desired reproduction end point when the RF data is loaded from said cine-memory (31) and a moving image is reproduced; and
a control unit (80) for outputting information for designating a range of address to be used when the RF data is loaded from said cine-memory (31) to said cine-memory reproducing unit (32) according to the reproduction start point and/or the reproduction end point set by using said input unit (70).

9. An ultrasonic diagnostic apparatus according to any one of claims 1-8, further comprising:
an RF data compressing unit (90) for compressing the RF data generated by said transmitting and receiving unit (20) according to a data compression method including difference coding processing; and
a communication unit (100) for transmitting the RF data compressed by said RF data compressing unit (90) with the information on frame rate to an external apparatus via a network.

10. A data analysis and measurement apparatus connected via a network to an ultrasonic diagnostic apparatus (2) according to claim 9 and a data accumulation apparatus (4) for accumulating compressed RF data with the information on frame rate, said data analysis and measurement apparatus comprising:
a communication unit (100) for receiving the compressed RF data with the information on frame rate from said data accumulation apparatus (4) via a network;
a data decompressing unit (110) for decompressing the compressed RF data received by said communication unit (100) ;
a cine-memory (31) for storing the RF data decompressed by said data decompressing unit (110) with the information on frame rate;
a cine-memory reproducing unit (32) for generating address information of the RF data in synchronization with a clock signal having a frequency controlled based on the information on frame rate stored in said cine-memory (31) when the RF data is loaded from said cine-memory (31);
a data analyzing and measuring unit (40) for performing analysis and/or measurement on a selected region of interest based on the RF data loaded from said cine-memory (31);
an image signal generating unit (50) for generating an image signal based on the RF data loaded from said cine-memory (31); and
a display unit (60) for displaying an ultrasonic image based on the image signal generated by said image signal generating unit (50).

11. A data analysis and measurement apparatus according to claim 10, further comprising:
a control unit (80) for controlling the frequency of said clock signal based on the information on frame rate stored in said cine-memory (31).

12. A data analysis and measurement apparatus according to claim 10 or 11, further comprising:
an input unit (70) to be used for setting a desired reproduction start point and/or a desired reproduction end point when the RF data is loaded from said cine-memory (31) and a moving image is reproduced; and
a control unit (80) for outputting information for designating a range of address to be used when the RF data is loaded from said cine-memory (31) to said cine-memory reproducing unit (32) according to the reproduction start point and/or reproduction end point set by using said input unit (70).

## Patentansprüche

1. Ultraschall-Diagnosevorrichtung, umfassend:
eine Ultraschallsonde (10) mit einer Mehrzahl von Ultraschallwandlern (10a) zum Aussenden von Ultraschallwellen auf ein zu prüfendes Objekt abhängig von Treibersignalen, und zum Empfangen von Ultraschallechos, die von dem Objekt reflektiert werden, um Detektorsignale auszugeben;
eine Sende- und Empfangseinheit (20) zum Liefern der Treibersignale an die Ultraschallsonde (10), zum Generieren von HF-Signalen basierend auf den Detektorsignalen, und zur A/D-Umsetzung der HF-Signale, um HF-Daten zu erzeugen;
einen Kinospeicher (31) zum Speichern der von der Sende- und Empfangseinheit (20) erzeugten HF-Daten mit Information bezüglich einer Bildfrequenz;
eine Kinospeicher-Wiedergabeeinheit (32);
eine Datenanalysier- und -messeinheit (40) zum Ausführen einer Analyse und/oder einer Messung in einer ausgewählten interessierenden Zone, basierend auf HF-Daten;
eine Bildsignal-Erzeugungseinheit (50) zum Generieren eines Bildsignals; und
eine Anzeigeeinheit (60) zum Anzeigen eines Ultraschallbilds basierend auf dem von der Bildsignal-Erzeugungseinheit (50) erzeugten Bildsignal, **dadurch gekennzeichnet, dass** die Kinospeicher-Wiedergabeeinheit (32) dazu ausgebildet ist, Adressinformation der HF-Daten synchron mit einem Taktsignal zu erzeugen, dessen Frequenz basierend auf der Information über die Bildfrequenz, die in dem Kinospeicher (31) gespeichert ist, zu erzeugen, wenn die HF-Daten aus dem Kinospeicher (31) geladen werden, dass die Datenanalysier- und -messeinheit (40) dazu ausgebildet ist, eine Analyse und/oder eine Messung selektiv entweder bezüglich der von der Sende- und Empfangseinheit (20) erzeugten HF-Daten auszuführen, oder bezüglich der aus dem Kinospeicher (31) geladenen HF-Daten, und die Bildsignal-Erzeugungseinheit (50) dazu ausgebildet ist, ein Bildsignal selektiv basierend auf den von der Sende- und Empfangseinheit (20) erzeugten HF-Daten oder den aus dem Kinospeicher (31) geladenen HF-Daten zu erzeugen.

2. Ultraschalldiagnosevorrichtung nach Anspruch 1, bei der die Sende-und Empfangseinheit (20) Verzögerungszeiten der Treibersignale einstellt und die Treibersignale derart an die Ultraschallsonde (10) liefert, dass die von den mehreren Ultraschallwandlern (10a) gesendeten Ultraschallwellen ein Ultraschallstrahlbündel bilden.

3. Ultraschalldiagnosevorrichtung nach Anspruch 1, bei der die Sende-und Empfangseinheit (20) die Treibersignale derart an die Ultraschallsonde (10) liefert, dass die mit einem Mal von den mehreren Ultraschallwandlern (10a) gesendeten Ultraschallwellen eine komplette Abbildungszone des Objekts erreichen.

4. Ultraschalldiagnosevorrichtung nach Anspruch 1, bei der die Sende-und Empfangseinheit (20) die HF-Signale **dadurch** erzeugt, dass sie die von den mehreren Ultraschallwandlern (10a) ausgegebenen Detektorsignale verstärkt, die Verzögerungszeiten der Detektorsignale einstellt und die Detektorsignale aufeinander addiert.

5. Ultraschalldiagnosevorrichtung nach Anspruch 1, bei der die Sende-und Empfangseinheit (20) die HF-Signale **dadurch** erzeugt, dass sie die Detektorsignale, die von den mehreren Ultraschallwandlern (10a) ausgegeben werden, verstärkt.

6. Ultraschalldiagnosevorrichtung nach Anspruch 3, bei der die Sende-und Empfangseinheit (20) die HF-Signale **dadurch** erzeugt, dass sie eine Wellenfrontsynthese ausführt basierend auf den Detektorsignalen, die durch Empfangen der Ultraschallechos zu unterschiedlichen Zeiten erhalten werden.

7. Ultraschalldiagnosevorrichtung nach einem der Ansprüche 1 bis 6, weiterhin umfassend:
eine Steuereinheit (80) zum Steuern der Frequenz der Taktsignale basierend auf der Information der Bildfrequenz, die in dem Kinospeicher (31) gespeichert ist.

8. Ultraschalldiagnosevorrichtung nach einem der Ansprüche 1 bis 6, weiterhin umfassend:
eine Eingabeeinheit (70) zur Verwendung beim Einstellen eines gewünschten Wiedergabe-Startpunkts und/oder eines gewünschten Wiedergabe-Endpunkts, wenn die HF-Daten aus dem Kinospeicher (31) geladen werden und ein bewegliches Bild wiedergegeben wird; und
eine Steuereinheit (80) zum Ausgeben von Information zum Kennzeichnen eines Adressenbereichs zur Verwendung dann, wenn die HF-Daten aus dem Kinospeicher (31) in die Kinospeicher-Wiedergabeeinheit (32) geladen werden, abhängig von dem Wiedergabestartpunkt und/oder den Wiedergabeendpunkt, der mit Hilfe der Eingabeeinheit (70) eingestellt wurde.

9. Ultraschalldiagnosevorrichtung nach einem der Ansprüche 1 bis 8, weiterhin umfassend:
eine HF-Daten-Kompressionseinheit (90) zum Komprimieren der von der Sende- und Empfangseinheit (20) erzeugten HF-Daten gemäß einem Datenkompressionsverfahren, welches die Differenz-Kodierverarbeitung beinhaltet; und
eine Kommunikationseinheit (100) zum Senden der von der HF-Daten-Kompressionseinheit (90) komprimierten HF-Daten mit der Information bezüglich der Bildfrequenz über ein Netzwerk an einer externen Vorrichtung.

10. Datenanalyse- und -messvorrichtung, die über ein Netzwerk an eine Ultraschalldiagnosevorrichtung (2) nach Anspruch 9 und eine Datensammelvorrichtung (4) zum Sammeln komprimierter HF-Daten mit der Information bezüglich der Bildfrequenz angeschlossen ist, wobei die Datenanalyse- und -messvorrichtung aufweist:
eine Kommunikationseinheit (100) zum Empfangen der komprimierten HF-Daten mit der Information bezüglich der Bildfrequenz von der Datensammelvorrichtung (4) über ein Netzwerk;
eine Datendekompressionseinheit (110) zum Dekomprimieren der von der Kommunikationseinheit (100) empfangenen, komprimierten HF-Daten;
einen Kinospeicher (31) zum Speichern der von der Datenkompressionseinheit (110) dekomprimierten HF-Daten mit der Information bezüglich der Bildfrequenz;
eine Kinospeicher-Wiedergabeeinheit (32) zum Erzeugen von Adressinformation der HF-Daten synchron mit einem Taktsignal, dessen Frequenz basierend auf der Information über die Bildfrequenz, die in den Kinospeicher (31) gespeichert ist, gesteuert wird, wenn die HF-Daten aus dem Kinospeicher (31) geladen werden;
eine Datenanalysier- und -messeinheit (40) zum Ausführen einer Analyse- und/oder einer Messung bezüglich einer ausgewählten interessierenden Zone, basierend auf den aus dem Kinospeicher (31) geladenen HF-Daten;
eine Bildsignalerzeugungseinheit (50) zum Erzeugen eines Bildsignals basierend auf den aus dem Kinospeicher (31) geladenen HF-Daten; und
eine Anzeigeeinheit (40) zum Anzeigen eines Ultraschallbilds basierend auf dem von der Bildsignal-Erzeugungseinheit (50) erzeugten Bildsignals.

11. Datenanalyse- und -messvorrichtung nach Anspruch 10, weiterhin umfassend:
eine Steuereinheit (80) zum Steuern der Frequenz des Taktsignals basierend auf der Information bezüglich der Bildfrequenz, die in dem Kinospeicher (31) gespeichert ist.

12. Datenanalyse- und -messvorrichtung nach Anspruch 10 oder 11, weiterhin umfassend:
eine Eingabeeinheit (70) zur Verwendung beim Einstellen eines gewünschten Wiedergabe-Startpunkts und/oder eines gewünschten Wiedergabe-Endpunkts, wenn die HF-Daten aus dem Kinospeicher (31) geladen werden und ein bewegliches Bild wiedergegeben wird; und
eine Steuereinheit (80) zum Ausgeben von Information zum Kennzeichnen eines Adressenbereichs zur Verwendung dann, wenn die HF-Daten aus dem Kinospeicher (31) in die Kinospeicher-Wiedergabeeinheit (32) geladen werden, abhängig von dem Wiedergabestartpunkt und/oder den Wiedergabeendpunkt, der mit Hilfe der Eingabeeinheit (70) eingestellt wurde.

## Revendications

1. Appareil de diagnostic ultrasonore comprenant :
une sonde ultrasonore (10) comprenant plusieurs transducteurs ultrasonores (10a) pour transmettre des ondes ultrasonores à un objet à inspecter conformément à des signaux de commande et recevoir les échos ultrasonores réfléchis par l'objet pour délivrer des signaux de détection ;
une unité d'émission et de réception (20) pour délivrer les signaux de commande à ladite sonde ultrasonore (10), générer des signaux radiofréquences sur la base des signaux de détection, et convertir d'analogique en numérique les signaux radiofréquences pour générer des données radiofréquences ;
une mémoire tournante (31) pour mémoriser les données radiofréquences générées par ladite unité d'émission et de réception (20) avec des informations concernant la fréquence de trame ;
une unité de reproduction de mémoire tournante (32) ;
une unité d'analyse et de mesure de données (40) pour effectuer une analyse et/ou une mesure sur une région d'intérêt sélectionnée sur la base des données radiofréquences ;
une unité de génération de signal d'image (50) pour générer un signal d'image ; et
une unité d'affichage (60) pour afficher une image ultrasonore sur la base du signal d'image généré par ladite unité de génération de signal d'image (50), **caractérisé en ce que** ladite unité de reproduction de mémoire tournante (32) est conçue pour générer des informations d'adresse des données radiofréquences en synchronisation avec un signal d'horloge ayant une fréquence commandée sur la base des informations concernant la fréquence de trame mémorisées dans ladite mémoire tournante (31) lorsque les données radiofréquences sont chargées à partir de ladite mémoire tournante (31), **en ce que** ladite unité d'analyse et de mesure de données (40) est conçue pour effectuer une analyse et/ou une mesure de manière sélective soit sur les données radiofréquences générées par ladite unité d'émission et de réception (20), soit sur les données radiofréquences chargées à partir de ladite mémoire tournante (31), et **en ce que** ladite unité de génération de signal d'image (50) est conçue pour générer un signal d'image de manière sélective sur la base des données radiofréquences générées par ladite unité d'émission et de réception (20) ou des données radiofréquences chargées à partir de ladite mémoire tournante (31).

2. Appareil de diagnostic ultrasonore selon la revendication 1, dans lequel ladite unité d'émission et de réception (20) ajuste les quantités de retard des signaux de commande et délivre les signaux de commande à ladite sonde ultrasonore (10) de sorte que les ondes ultrasonores transmises par ladite pluralité de transducteurs ultrasonores (10a) forment un faisceau ultrasonore.

3. Appareil de diagnostic ultrasonore selon la revendication 1, dans lequel ladite unité d'émission et de réception (20) délivre les signaux de commande à ladite sonde ultrasonore (10) de sorte que les ondes ultrasonores transmises simultanément par ladite pluralité de transducteurs ultrasonores (10a) atteignent une région d'imagerie entière de l'objet.

4. Appareil de diagnostic ultrasonore selon la revendication 1, dans lequel ladite unité d'émission et de réception (20) génère les signaux radiofréquences en amplifiant les signaux de détection délivrés par ladite pluralité de transducteurs ultrasonores (10a), en ajustant les quantités de retard des signaux de détection, et en additionnant les signaux de détection les uns avec les autres.

5. Appareil de diagnostic ultrasonore selon la revendication 1, dans lequel ladite unité d'émission et de réception (20) génère les signaux radiofréquences en amplifiant les signaux de détection délivrés par ladite pluralité de transducteurs ultrasonores (10a).

6. Appareil de diagnostic ultrasonore selon la revendication 3, dans lequel ladite unité d'émission et de réception (20) génère les signaux radiofréquences en effectuant une synthèse de front d'onde sur la base des signaux de détection obtenus en recevant les échos ultrasonores à plusieurs instants différents.

7. Appareil de diagnostic ultrasonore selon l'une quelconque des revendications 1 à 6, comprenant en outre :
une unité de commande (80) pour commander la fréquence dudit signal d'horloge sur la base des informations concernant la fréquence de trame mémorisées dans ladite mémoire tournante (31).

8. Appareil de diagnostic ultrasonore selon l'une quelconque des revendications 1 à 6, comprenant en outre :
une unité d'entrée (70) destinée à être utilisée pour fixer un point de début de reproduction souhaité et/ou un point de fin de reproduction souhaité lorsque les données radiofréquences sont chargées à partir de ladite mémoire tournante (31) et qu'une image animée est reproduite ; et
une unité de commande (80) pour délivrer des informations pour désigner une plage d'adresses à utiliser lorsque les données radiofréquences sont chargées à partir de ladite mémoire tournante (31) à ladite unité de reproduction de mémoire tournante (32) conformément au point de début de reproduction et/ou au point de fin de reproduction fixés en utilisant ladite unité d'entrée (70).

9. Appareil de diagnostic ultrasonore selon l'une quelconque des revendications 1 à 8, comprenant en outre :
une unité de compression de données radiofréquences (90) pour compresser les données radiofréquences générées par ladite unité d'émission et de réception (20) selon un procédé de compression de données comprenant un traitement de codage différentiel ; et
une unité de communication (100) pour transmettre les données radiofréquences compressées par ladite unité de compression de données radiofréquences (90) avec les informations concernant la fréquence de trame à un appareil externe par l'intermédiaire d'un réseau.

10. Appareil d'analyse et de mesure de données connecté par l'intermédiaire d'un réseau à un appareil de diagnostic ultrasonore (2) selon la revendication 9 et un appareil d'accumulation de données (4) pour accumuler les données radiofréquences compressées avec les informations concernant la fréquence de trame, ledit appareil d'analyse et de mesure de données comprenant :
une unité de communication (100) pour recevoir les données radiofréquences compressées avec les informations concernant la fréquence de trame dudit appareil d'accumulation de données (4) par l'intermédiaire d'un réseau ;
une unité de décompression de données (110) pour décompresser les données radiofréquences compressées reçues par ladite unité de communication (100) ;
une mémoire tournante (31) pour mémoriser les données radiofréquences décompressées par ladite unité de décompression de donnés (110) avec les informations concernant la fréquence de trame ;
une unité de reproduction de mémoire tournante (32) pour générer les informations d'adresse des données radiofréquences en synchronisation avec un signal d'horloge ayant une fréquence commandée sur la base des informations concernant la fréquence de trame mémorisées dans ladite mémoire tournante (31) lorsque les données radiofréquences sont chargées à partir de ladite mémoire tournante (31) ;
une unité d'analyse et de mesure de données (40) pour effectuer une analyse et/ou une mesure sur une région d'intérêt sélectionnée sur la base des données radiofréquences chargées à partir de ladite mémoire tournante (31) ;
une unité de génération de signal d'image (50) pour générer un signal d'image sur la base des données radiofréquences chargées à partir de ladite mémoire tournante (31) ; et
une unité d'affichage (60) pour afficher une image ultrasonore sur la base du signal d'image généré par ladite unité de génération de signal d'image (50).

11. Appareil d'analyse et de mesure de données selon la revendication 10, comprenant en outre :
une unité de commande (80) pour commander la fréquence dudit signal d'horloge sur la base des informations concernant la fréquence de trame mémorisées dans ladite mémoire tournante (31).

12. Appareil d'analyse et de mesure de données selon la revendication 10 ou 11, comprenant en outre :
une unité d'entrée (70) destinée à être utilisée pour fixer un point de début de reproduction souhaité et/ou un point de fin de reproduction souhaité lorsque les données radiofréquences sont chargées à partir de ladite mémoire tournante (31) et qu'une image animée est reproduite ; et
une unité de commande (80) pour délivrer des informations pour désigner une plage d'adresses à utiliser lorsque les données radiofréquences sont chargées à partir de ladite mémoire tournante (31) à ladite unité de reproduction de mémoire tournante (32) conformément au point de début de reproduction et/ou au point de fin de reproduction fixés en utilisant ladite unité d'entrée (70).
